(19) Europäisches Patentamt
European Patent Office
Office européen des brevets

(11) **EP 3 296 717 B1**

(12) **EUROPÄISCHE PATENTSCHRIFT**

(45) Veröffentlichungstag und Bekanntmachung des Hinweises auf die Patenterteilung:
**10.03.2021   Patentblatt 2021/10**

(21) Anmeldenummer: **17186367.3**

(22) Anmeldetag: **16.08.2017**

(51) Int Cl.:
*G01N 21/03* (2006.01)     *G01N 21/25* (2006.01)
*G01N 21/53* (2006.01)     *G01N 21/85* (2006.01)
*A61M 1/36* (2006.01)     *G01N 21/64* (2006.01)
*G01N 21/88* (2006.01)     *G01N 21/94* (2006.01)
*G01N 21/17* (2006.01)

(54) **ERFASSUNGSVORRICHTUNG FÜR EIN MEDIUM IN EINEM SCHLAUCHABSCHNITT**

DETECTION DEVICE FOR A MEDIUM IN A HOSE SECTION

DISPOSITIF DE DÉTECTION D'UN MILIEU DANS UN TRONÇON DE FLEXIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.08.2016   DE 102016116100**

(43) Veröffentlichungstag der Anmeldung:
**21.03.2018   Patentblatt 2018/12**

(73) Patentinhaber: **B. Braun Avitum AG**
**34212 Melsungen (DE)**

(72) Erfinder: **STROHHÖFER, Christof, Dr.**
**1020 Wien (AT)**

(74) Vertreter: **Winter, Brandl, Fürniss, Hübner, Röss, Kaiser, Polte - Partnerschaft mbB Patent- und Rechtsanwaltskanzlei Alois-Steinecker-Straße 22 85354 Freising (DE)**

(56) Entgegenhaltungen:
**EP-A1- 0 467 804     EP-A1- 2 264 512 WO-A1-00/39573**

EP 3 296 717 B1

**Beschreibung**

Die Erfindung betrifft eine Erfassungsvorrichtung für ein Medium in einem Schlauchabschnitt und bezieht sich insbesondere auf eine Erfassungsvorrichtung der Art, die ein Beleuchtungssystem für abbildende Sensoren an medizinischen Geräten, beispielsweise solchen, bei welchen Blutschlauchsysteme zur Anwendung kommen, beinhaltet.

In der Dialyse fließt das Blut des Patienten durch ein Schlauchsystem eines extrakorporalen Blutkreislaufs. Einerseits ermöglicht das extrakorporale Führen von Blut einen leichten Zugang zu dem Blut des Patienten, um daran analytische Parameter zu bestimmen. Andererseits ist das extrakorporale Führen des Bluts ein Risikofaktor in der Dialysebehandlung, da dadurch dem Patienten unerwünschte Fremdstoffe wie Verunreinigungen oder Luft zugeführt werden können.

Entsprechend vielfältig sind die Einsatzmöglichkeiten verschiedenster Sensorikanwendungen, die auf die Erfassung solcher Fremdstoffe abzielen. Besonderes Augenmerk liegt dabei auf Sensorverfahren, welche das Blut bzw. den Inhalt des Blutschlauchsystems berührungslos und zerstörungsfrei analysieren können. Berührungslos bedeutet in diesem Zusammenhang, dass das Blut nicht mit einem anderen Material als dem Standardmaterial des Schlauchsystems in Berührung kommt. Damit wird zum einen das Auftreten von zusätzlichen Koagulations- oder Entzündungsherden vermieden. Zum anderen werden Herstellungskosten gering gehalten, da keine Kombination von unterschiedlichen Materialien und Strukturen erfolgen muss. Zerstörungsfrei bedeutet in diesem Zusammenhang, dass die Messmethode keinen negativen Einfluss auf die zellulären oder molekularen Bestandteile des Blutes ausübt, diese schwächt oder gar zerstört. Dies ist besonders wichtig, da bei Dialysepatienten die Bildung von roten Blutkörperchen beeinträchtigt ist und jeder Verlust derselben den Gesundheitszustand und das Wohlbefinden des Patienten dauerhaft beeinträchtigen kann.

Es ist bekannt, dass sich optische Technologien für die berührungslose und zerstörungsfreie Analyse von Stoffen eignen. Licht kann ohne direkten Kontakt zwischen Analyt und Lichtquelle oder Detektor mit dem Analyten wechselwirken und führt nur unter extremen Bedingungen zu einer Schädigung von beispielsweise Zellen. Diese extremen Bedingungen lassen sich jedoch leicht durch geschicktes Design des Sensors vermeiden.

Der Einsatz von optischen Sensoren in der Dialyse ist bekannt. Hierzu zählen z.B. Hämatokritsensoren (basierend auf rotem und infrarotem Licht), Rotdetektoren (zeigen die Präsenz von Blut im Blutschlauchsystem an) und Blutleckdetektoren (messen eine Rotfärbung in Dialysierflüssigkeit im Dialysierflüssigkeitskreislauf) oder analytische Sensoren im Dialysierflüssigkeitskreislauf für Echtzeitmonitoring, beispielsweise solchen, die eine Bestimmung einer Dialysedosis während der Therapie erlauben. Diese Sensoren messen zumindest Quasi-Gleichgewichtszustände, d.h. sie geben beispielsweise eine Konzentration, eine Färbung oder die Anwesenheit eines Stoffes im Blut an. Dazu wird die Gesamtheit des auf den Detektor auftreffenden Lichts analysiert und zu einem Messwert verarbeitet. Aus diesem Grund werden keine besonderen Ansprüche an das Beleuchtungssystem dieser Sensoren gestellt. Diese Sensoren können im Allgemeinen aber keine Aussagen über kurzfristige Störungen treffen, bzw. werden von diesen sogar negativ beeinflusst, und sind auch nicht in der Lage, einen Messwert ortsaufgelöst, d.h. an verschiedenen Punkten im Inneren des Schlauchs, zu bestimmen. EP2264512 A1 offenbart ein Mikroskop zur Abbildung von extrakorporalem Blut in einem Schlauch mit einer Vorrichtung zur gleichmässigen Ausleuchtung der Probe.

In einigen Anwendungen, wie beispielsweise der Detektion von Luftblasen oder gelösten Verunreinigungen, ist jedoch eine Ortsauflösung günstig, um diese Luftblasen oder Verunreinigungen zu identifizieren und zu quantifizieren (Luftblasen z.B. nach Volumen, Verunreinigungen nach Größe und Struktur). Für eine ortsaufgelöste Messung werden Detektorarrays benötigt, welche durch die Kombination einer Vielzahl an Pixeln räumliche Informationen wiedergeben können. Ein einfaches Beispiel für ein solches Messsystem mit Ortsauflösung sind Kameras, die das zu untersuchende System auf ein CMOS- oder CCD-Element abbilden und so die räumliche Information zur Analyse bereitstellen. Anders als bei optischen Sensoren ist bei solchen abbildenden Messsystemen eine homogene Ausleuchtung des gesamten Detektorarrays eine wichtige Eigenschaft. Ist ein Pixel des Detektorarrays schwächer ausgeleuchtet als ein anderes, wird dies auf eine lokale Wechselwirkung zwischen Licht und Analyt zurückgeführt. Ist ein Ausleuchtungsunterschied anstelle dessen durch eine inhomogene Verteilung des einfallenden Lichts verursacht, führt dies zu einer Fehlinterpretation der Messung.

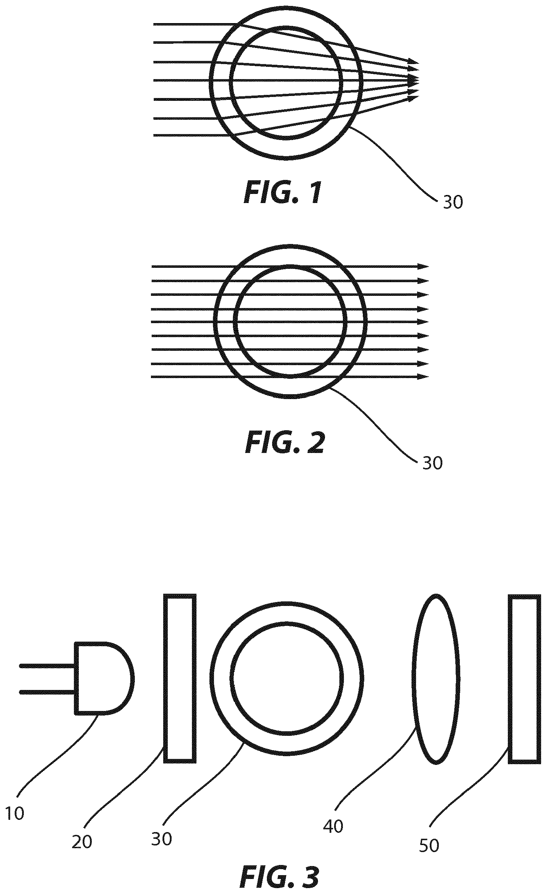

Bei einer Messung an einem Schlauch sind weitere Kriterien zu beachten. Ein Schlauch als ein im Wesentlichen zylinderförmiges Objekt kann zum einen als Linse wirken, mit der Folge einer unterschiedlichen Ausleuchtung des Detektorarrays, wie in Fig. 1 schematisch dargestellt. Der in Fig. 1 gezeigte Linseneffekt führt bei Durchstrahlung eines Schlauchs zu inhomogener Lichtverteilung an einer Detektoreinrichtung. Der Effekt hängt zudem davon ab, ob und welches Fluid sich darin befindet.

Zum anderen sind bei einer Durchstrahlung des Schlauchquerschnitts die optischen Wege des Lichts durch das Medium im Schlauch unterschiedlich (vgl. Fig. 2). Auch dies führt in der Regel zu einer inhomogenen Ausleuchtung des Detektorarrays, da bei homogener Durchstrahlung eines Schlauchs die Lichtstrahlen am Rand des Schlauchs weniger stark mit dem Medium im Schlauch interagieren als Lichtstrahlen im Mittenbereich des Schlauchs. Dies führt ebenfalls zu inhomogener Lichtverteilung auf der Detektoreinrichtung, da Licht, das am Rand des Schlauchs durchdringt, weniger absorbiert wird als Licht, das durch den Mittenbereich des Schlauchs hindurchtritt. Dazu analoge Ausleuchtungsunterschiede können auftreten, wenn das Beleuchtungssystem aus einer Punktlichtquelle oder wenigen Punktlichtquellen, wie beispielsweise Anordnungen von Leuchtdioden bzw. LEDs, besteht.

Wie vorstehend beschrieben wurde, ist im Stand der Technik bislang lediglich eine homogene Ausleuchtung einer ebenen Fläche angesprochen.

Der Erfindung liegt daher als eine Aufgabe zugrunde, eine Erfassungsvorrichtung für ein Medium in einem Schlauchabschnitt zu schaffen, die auf der Grundlage einer homogenen Ausleuchtung des Innenraums des Schlauchabschnitts eine gute Abbildung des Schlauchinnenraums mittels abbildender und/oder ortsaufgelöster optischer Verfahren erlaubt.

Erfindungsgemäß wird diese Aufgabe durch eine Erfassungsvorrichtung für ein Medium in einem Schlauchabschnitt mit den Merkmalen des Anspruchs 1 gelöst. Vorteilhafte Weiterbildungen sind Gegenstand der beigefügten Unteransprüche.

Der Erfindung liegt als eine allgemeine Idee zugrunde, homogen verteiltes Licht für einen abbildenden optischen Sensor bereitzustellen. Der Sensor bildet zumindest Teil einer Detektoreinrichtung, welche ein Medium in einem Schlauch oder zumindest Schlauchabschnitt analysiert. Das bereitgestellte Licht ist geeignet homogenisiert, so dass Helligkeitsunterschiede, die auf lokale Unterschiede des Mediums in dem Schlauch oder Schlauchabschnitt zurückzuführen sind, erkannt und quantifiziert (ausgewertet) werden können. Die lokalen Unterschiede können beispielsweise Dichteänderungen eines absorbierenden Stoffs, Einschlüsse anderer Medien (z.B. Luft in einer Flüssigkeit), Feststoffpartikel in einer Flüssigkeit oder dergleichen sein. Der Schlauch oder Schlauchabschnitt kann entweder zwischen einer Lichtquelle und einer Detektoreinrichtung (Detektorarray) angeordnet sein, oder es können die Lichtquelle und die Detektoreinrichtung auf derselben Schlauchseite angeordnet sein und/oder in einem vorbestimmten Winkel zueinander stehen.

Eine Homogenisierungsvorrichtung für das Licht ist zwischen der Lichtquelle und dem Schlauch angeordnet, so dass der Schlauch von der Lichtquelle indirekt beleuchtet wird. Als Homogenisierungsvorrichtung kann beispielsweise eine Streuplatte, eine Diffusorfolie oder allgemein ein transparentes, aufgerautes Medium verwendet werden. Das vorstehend beschriebene Grundprinzip, bei dem im Kern anstelle einer direkten Beleuchtung des Schlauchs durch eine Lichtquelle eine Homogenisierungsvorrichtung für das Licht zwischen Lichtquelle und Schlauch platziert ist, ist schematisch in Fig. 3 wiedergegeben.

Speziell vorteilhafte Ausführungsformen der Homogenisierungsvorrichtung umfassen zumindest teilweise konvexe und/oder konkave Oberflächen und/oder eine variable Absorptionsfunktion, d.h. Kombinationen von Streu- und/oder Diffusorkörpern mit einem absorbierenden Material, durch stufenweise oder graduell verlaufende Einbringung absorbierender Substanzen in vorbestimmte Bereiche der Homogenisierungsvorrichtung senkrecht zu der Lichtausbreitungsrichtung. Erfindungsgemäß beinhaltet die variable Absorptionsfunktion eine stärkere Lichtabsorption in vorbestimmten Außenbereichen oder Randbereichen der Homogenisierungsvorrichtung, wobei die Lichtabsorption zu einem Mittenbereich der Homogenisierungsvorrichtung hin schwächer wird.

Im Einzelnen wird die Aufgabe gelöst durch eine Erfassungsvorrichtung für ein Medium in einem Schlauchabschnitt, beinhaltend: eine abbildende Optik, einen Schlauchabschnitt, zumindest eine Lichtquelle, die an einer ersten Position in Bezug auf einen von Licht durchstrahlbaren Schlauchabschnitt angeordnet und dazu konfiguriert ist, Licht auf den Schlauchabschnitt abzustrahlen; eine Detektoreinrichtung, die an einer zweiten Position in Bezug auf den Schlauchabschnitt angeordnet und dazu konfiguriert ist, durch den Schlauchabschnitt hindurch gelangtes Licht von der zumindest einen Lichtquelle zu empfangen und ein Medium in dem Schlauchabschnitt ortsauflösend zu analysieren; und eine Homogenisierungsvorrichtung, die an einer Position zwischen der zumindest einen Lichtquelle und dem Schlauchabschnitt angeordnet und dazu konfiguriert ist, zur Erfassung und Quantisierung von Helligkeitsunterschieden aufgrund lokaler Unterschiede des Mediums in dem Schlauchabschnitt eine homogene und/oder isotrope Verteilung des Lichts von der zumindest einen Lichtquelle zu erzeugen, bevor das Licht in den Schlauchabschnitt eintritt, wobei die Homogenisierungsvorrichtung entlang einer Richtung senkrecht zu der Durchstrahlungsrichtung in vorbestimmte Bereiche der Homogenisierungsvorrichtung durch gezielte Veränderung des Basismaterials und/ oder durch Kombination unterschiedlicher Materialien vorbestimmte Konzentrationen Licht absorbierender Substanzen aufweist, die eine veränderlich Licht absorbierende Funktion der Homogenisierungsvorrichtung bereitstellen,dadurch gekennzeichnet, dass die Homogenisierungsvorrichtung in einem durch zumindest die abbildende Optik, deren Öffnungswinkel und deren Fokusbereich bestimmten kleinsten Abstand zu dem Schlauchabschnitt angeordnet ist; eine Lichtleitvorrichtung, die eine erste und eine zweite Lichtleitvorrichtung umfasst und die in zumindest dem Bereich zwischen der Homogenisierungsvorrichtung und dem Schlauchabschnitt als eine Wandung konfiguriert ist, welche zumindest den Bereich ummantelnd umgibt, wobei die zumindest eine Lichtquelle und/oder die Homogenisierungsvorrichtung zwischen der ersten und der zweiten Lichtleitvorrichtung angeordnet ist/sind; und die vorbestimmten Konzentrationen Licht absorbierender Substanzen in die Homogenisierungsvorrichtung derart integriert sind, dass in einem oberen und in einem unteren Außenbereich der Homogenisierungsvorrichtung die Konzentration der beigefügten Substanzen jeweils höher und in einem zentralen Bereich oder Mittenbereich demgegenüber geringer ist, wobei der obere und der untere Außenbereich der Homogenisierungsvorrichtung sowie deren zentraler Bereich oder Mittenbereich entlang der Richtung senkrecht zu der Durchstrahlungsrichtung angeordnet sind, und ein dadurch entstehendes Lichtabsorptionsprofil an geometrische Gegebenheiten des Schlauchabschnitts und die optischen Eigenschaften des Mediums angepasst ist.

Bevorzugt ist in einer geometrischen Anordnung für eine Transmissionsmessung die zumindest eine Lichtquelle derart auf einer ersten Seite des Schlauchabschnitts angeordnet und die Detektoreinrichtung derart auf einer zweiten Seite des Schlauchabschnitts angeordnet, dass sich der Schlauchabschnitt zwischen der zumindest einen Lichtquelle und der Detektoreinrichtung befindet.

Alternativ bevorzugt sind in einer geometrischen Anordnung für eine Reflexionsmessung die zumindest eine Lichtquelle und die Detektoreinrichtung bezüglich des Schlauchabschnitts auf derselben Seite angeordnet und diese nehmen dabei einen vorbestimmten Winkel zueinander ein.

Bevorzugt ist die Homogenisierungsvorrichtung eine Streuplatte, eine Diffusorfolie oder ein transparentes Medium mit aufgerauter Oberfläche.

Es sind zumindest eine erste und eine zweite Wandung vorgesehen, zwischen welchen die zumindest eine Lichtquelle und/oder die Homogenisierungsvorrichtung angeordnet ist/sind. Konfigurationsbezogen können dabei die erste und/oder die zweite Wandung reflektierend ausgebildet sein, um eventuelle Lichtverluste entlang des Lichtwegs zu verringern, oder nicht reflektierend und/oder in einer vorbestimmten Farbgebung, beispielsweise schwarz, ausgebildet sein, um unerwünschte Reflexionen an etwa einer glattflächigen Oberfläche eines Schlauchs aus beispielsweise einem Kunststoffmaterial zu vermeiden.

Es ist eine abbildende Optik vor der Detektoreinrichtung und/oder zwischen der zumindest einen Lichtquelle und der Homogenisierungsvorrichtung angeordnet. Bevorzugt stimmt eine Größe der Homogenisierungsvorrichtung mit zumindest der Größe eines Sichtfelds der Detektoreinrichtung überein. Dabei kann sich bei einer gegebenen Flussrate des Mediums in dem Schlauchabschnitt und einer gegebenen Aufnahmerate der Detektorvorrichtung ein Sichtfeld der Detektoreinrichtung und eine minimale Abmessung der Homogenisierungsvorrichtung in Längsrichtung des Schlauchabschnitts zu GF = FR/AR bestimmen, und kann eine minimale Abmessung der Homogenisierungsvorrichtung in Querrichtung des Schlauchabschnitts zumindest gleich dem Gesamtdurchmesser des Schlauchabschnitts sein.

Bevorzugt ist die zumindest eine Lichtquelle dazu konfiguriert, eine Ausdehnung der Homogenisierungsvorrichtung in Längsrichtung des Schlauchabschnitts vollständig auszuleuchten, wobei für eine vollständige Ausleuchtung eine dazu angepasste Form der zumindest einen Lichtquelle oder eine Vielzahl von Lichtquellenelementen und/oder strahlformenden Elementen in einem Lichtweg vorgesehen sind.

Es sind in die Homogenisierungsvorrichtung entlang einer Richtung senkrecht zu der Durchstrahlungsrichtung bzw. Lichtausbreitungsrichtung in vorbestimmte Bereiche der Homogenisierungsvorrichtung vorbestimmte Konzentrationen eines Licht absorbierenden Stoffs eingebracht, die eine veränderlich Licht absorbierende Funktion der Homogenisierungsvorrichtung bereitstellen. Vorteilhaft können solche Konzentrationen stufenweise oder graduell veränderlich eingebracht sein und insoweit eine veränderliche Absorptionsfunktion für Licht der Homogenisierungsvorrichtung bereitstellen, mit welcher ungleiche optische Pfadlängen in dem Medium innerhalb des Schlauchs oder Schlauchabschnitts und eine daraus resultierende ungleichmäßige Beleuchtung oder Ausleuchtung eines Detektorarrays kompensierbar, zumindest aber signifikant verringerbar sind.

Bevorzugt kann ein medizinisches Gerät vorteilhaft eine Erfassungsvorrichtung wie vorstehend beschrieben beinhalten. Weiter vorteilhaft kann ein medizinisches Gerät als eine Maschine zur extrakorporalen Blutbehandlung, beispielsweise als eine Dialysemaschine, ausgebildet sein.

Die Erfindung wird nachstehend anhand bevorzugter Ausführungsbeispiele unter Bezugnahme auf die Zeichnung näher beschrieben. Es zeigen:

Fig. 1 schematisch einen Linseneffekt, der bei Durchstrahlung eines Schlauches zu inhomogener Lichtverteilung an einer Detektoreinrichtung führt;

Fig. 2 schematisch eine Interaktion von Strahlen am Rand und in einem Mittenbereich eines Schlauchs bei homogener Durchstrahlung des Schlauchs, wobei Randstrahlen weniger stark mit einem Medium in dem Schlauch interagieren als Zentralstrahlen und aufgrund unterschiedlicher Lichtabsorption am Rand und im Mittenbereich eine inhomogene Lichtverteilung auf der Detektoreinrichtung resultiert;

Fig. 3 vereinfacht und schematisch einen Aufbau einer Erfassungsvorrichtung zur lokal aufgelösten Analyse eines Mediums in einem Schlauch oder Schlauchabschnitt gemäß einem ersten Ausführungsbeispiel, das geometrisch für eine Transmissionsmessung konfiguriert ist;

Fig. 4 veranschaulichend eine Verbesserung der Ausleuchtung, die durch eine Homogenisierungsvorrichtung erhalten wird;

Fig. 5 vereinfacht und schematisch einen Aufbau einer Erfassungsvorrichtung zur lokal aufgelösten Analyse eines Mediums in einem Schlauch oder Schlauchabschnitt gemäß einem zweiten Ausführungsbeispiel, bei dem eine Lichtleitvorrichtung dazu angeordnet ist, einer Verringerung der Beleuchtungsintensität nach der Homogenisierungseinrichtung entgegenzuwirken;

Fig. 6 vereinfacht und schematisch einen Aufbau einer Erfassungsvorrichtung zur lokal aufgelösten Analyse eines Mediums in einem Schlauch oder Schlauchabschnitt gemäß einem dritten Ausführungsbeispiel, das geometrisch für eine Reflexionsmessung konfiguriert ist;

Fig. 7 eine in einer Modifikation der ersten bis dritten Ausführungsbeispiele verwendbare Ausgestaltung der Homogenisierungsvorrichtung in plano-konkaver Form;

Fig. 8 eine in einer alternativen Modifikation der ersten bis dritten Ausführungsbeispiele verwendbare Ausgestaltung der Homogenisierungsvorrichtung in plano-konvexer Form; und

Fig. 9 eine in einer weiteren alternativen Modifikation der ersten bis dritten Ausführungsbeispiele verwendbare Ausgestaltung der Homogenisierungsvorrichtung mit erhöhter Absorption in einem Außenbereich und geringerer Absorption in einem Mittenbereich.

Es wird angemerkt, dass in der Zeichnung gleiche oder gleichwirkende Elemente und Komponenten mit denselben Bezugszeichen bezeichnet sind und nicht redundant beschrieben werden.

Fig. 3 zeigt schematisch und vereinfacht einen Aufbau einer Erfassungsvorrichtung in Form eines optischen Sensors zur lokal aufgelösten Analyse eines Mediums in einem Schlauch oder Schlauchabschnitt gemäß einem ersten Ausführungsbeispiel.

In dem ersten Ausführungsbeispiel wird eine Transmissionsgeometrie verwendet, bei welcher eine Lichtquelle 10, beispielsweise eine Leuchtdiode bzw. LED, einen ein Medium führenden Schlauch oder Schlauchabschnitt 30 durchstrahlt und Licht von der Lichtquelle 10 nach Hindurchtreten durch den Schlauch oder Schlauchabschnitt 30 auf ein Detektorarray 50 trifft. Das Detektorarray 50 ist in diesem Ausführungsbeispiel als eine Erfassungs-, Sensor oder Detektoreinrichtung zu verstehen, die zumindest ein Erfassungs- oder Sensorelement aufweist. Insbesondere kann in diesem Ausführungsbeispiel das Detektorarray 50 als Bildaufnahmeeinrichtung und/oder Bildverarbeitungseinrichtung ausgebildet und zumindest dazu angepasst sein, bildhaft auswertbare Erfassungssignale zu erzeugen. Vor dem Detektorarray 50 ist eine abbildende Optik 40, beispielsweise eine Linsenanordnung, ein Kameraobjektiv und dergleichen, angeordnet:

Die Lichtquelle 10 kann in Abhängigkeit von einer entsprechenden Anwendung aus beispielsweise Lampen, wie etwa Deuterium-Lampen oder Hg-Lampen für Messaufgaben im UV-Bereich, Halogenlampen für Messaufgaben im Bereich sichtbaren und infraroten Lichts und/oder Leuchtdioden bzw. LEDs, welche entweder ein breites Emissionsspektrum haben können und dadurch nahezu weißes Licht emittieren oder nur in einem schmalen Band um eine bestimmte Wellenlänge Licht aussenden, ausgewählt sein. Insbesondere LEDs sind mit Lichtemission sowohl im UV-Bereich als auch im sichtbaren und infraroten Spektralbereich anwendbar. Ferner können Laser zur Anwendung kommen, die sehr schmalbandiges Licht vom UV-Bereich über den Bereich sichtbaren Lichts bis in den Infrarotbereich hinein emittieren können.

Um einen ausreichenden Grad an homogener Ausleuchtung zu erreichen, ist ferner eine Homogenisierungsvorrichtung 20 für das Licht von der Lichtquelle 10 zwischen der Lichtquelle 10 und dem Schlauchabschnitt 30 angeordnet.

Die Homogenisierungsvorrichtung 20 ist angeordnet, damit das Licht eines in diesem Ausführungsbeispiel beispielsweise als Lichtquelle 10 verwendeten Punktlichtstrahlers vor seinem Hindurchtreten durch den Schlauch oder Schlauchabschnitt 30 seine Vorzugsrichtung im Raum verliert und homogen und isotrop auf den Schlauch oder Schlauchabschnitt 30 abgestrahlt wird. Der Einsatz der Homogenisierungsvorrichtung 20 ist insbesondere in Spektralbereichen sinnvoll, in welchen Wellenlängen klein gegenüber den geometrischen Abmessungen des Sichtfelds sind.

Ferner optional kann eine (nicht dargestellte) Linsenanordnung zwischen der Lichtquelle 10 und der Homogenisierungsvorrichtung 20 die Lichtabstrahlung der Lichtquelle 10 in vorbestimmter Weise ändern, bevor ihr Licht auf die Homogenisierungsvorrichtung 20 trifft.

Bevorzugte Abmessungen und insgesamt eine Größe der Homogenisierungsvorrichtung 20 leiten sich aus Randbedingungen einer jeweiligen Anwendung ab. Ausschlaggebender Faktor ist hierbei jeweils das Bildfeld beteiligter Komponenten, wobei grundsätzlich die Abmessungen der Homogenisierungsvorrichtung 20 zumindest mit den Abmessungen des Sichtfelds des Detektorarrays 50 übereinstimmen sollten. Im Falle einer In-Fluss-Messung, d.h. einer Messung an einem fließenden Medium, ist darüber hinaus auch eine Geschwindigkeit der Bildaufnahme durch das Detektorarray 50 einzubeziehen.

Beispielsweise kann in einem die Konfiguration dieses Ausführungsbeispiels beinhaltenden System Blut mit einer Geschwindigkeit FR durch den Schlauch oder Schlauchabschnitt 30 gepumpt werden, und soll in diesem Fall die Erfassungsvorrichtung als Detektionssystem Luftblasen im Blut identifizieren und vermessen. Das Detektionssystem hat beispielsweise eine Aufnahmerate AR, d.h. es kann eine vorbestimmte Anzahl, z.B. N, von Aufnahmen pro Zeiteinheit, z.B. pro Sekunde, erzeugt werden.

In dem vorgenannten beispielhaften Fall bedeutet dies, dass in Intervallen von 1/N Sekunden jeweils eine Aufnahme erfolgen kann. Um sicherzustellen, dass keine Luftblase unentdeckt bleibt, muss das System so ausgelegt sein, dass bei gegebener Flussrate des Bluts eine Luftblase für mindestens 1/N Sekunden im Sichtfeld des erfassenden Sensors verbleibt. Für das Sichtfeld des erfassenden Sensors GF (längs des Schlauchs) resultiert daraus gemäß der nachstehenden Gleichung (1):

$$GF= FR / AR \qquad\qquad\qquad (1)$$

Dies ist gleichzeitig die minimale Abmessung der Homogenisierungsvorrichtung 20 in Längsrichtung des Schlauchs oder Schlauchabschnitts 30. Außerdem muss in Querrichtung des Schlauchs oder Schlauchabschnitts 30 die Abmessung der Homogenisierungseinrichtung 20 gleich oder größer als der Gesamtdurchmesser des Schlauchs oder Schlauchabschnitts 30 sein. Aufgrund der eingangs unter Bezugnahme auf Fig. 1 und Fig. 2 beschriebenen Effekte genügt eine Abmessung der Homogenisierungsvorrichtung 20 lediglich entsprechend dem Innendurchmesser des Schlauchs oder Schlauchabschnitts 30 nicht.

Die Abmessungen der Homogenisierungsvorrichtung 20 bzw. die Größe des Sichtfelds bestimmen im Weiteren die Ausführungsform der Lichtquelle 10 vor. Idealerweise wird die gesamte Homogenisierungsvorrichtung 20 von Licht bestrahlt. Insbesondere bei In-Fluss-Messsystemen kann die Dimensionierung entlang des Schlauchs oder Schlauchabschnitts 30 deutlich größer sein als die Dimensionierung quer zu dem Schlauch oder Schlauchabschnitt 30.

Die Lichtquelle 10 muss somit dazu angepasst sein, eine längliche Homogenisierungseinrichtung 20, d.h. eine Homogenisierungsvorrichtung 20, die länger ist als breit, vollständig auszustrahlen oder auszuleuchten. Um dies zu erreichen, können in diesem Ausführungsbeispiel dazu spezielle Formen der Lichtquelle 10, die ferner mit strahlformenden Elementen kombiniert sein können, oder eine Reihe aus mehreren kleineren oder punktförmigen Lichtquellen (Punktstrahler) vorgesehen sein. Bevorzugt sind im Fall kleinerer oder punktförmiger Lichtquellen diese so angeordnet, dass sich ihre Strahlungsfelder in der Ebene der Homogenisierungsvorrichtung 20 ausreichend überlappen, um eine vollständige Ausleuchtung sicherzustellen.

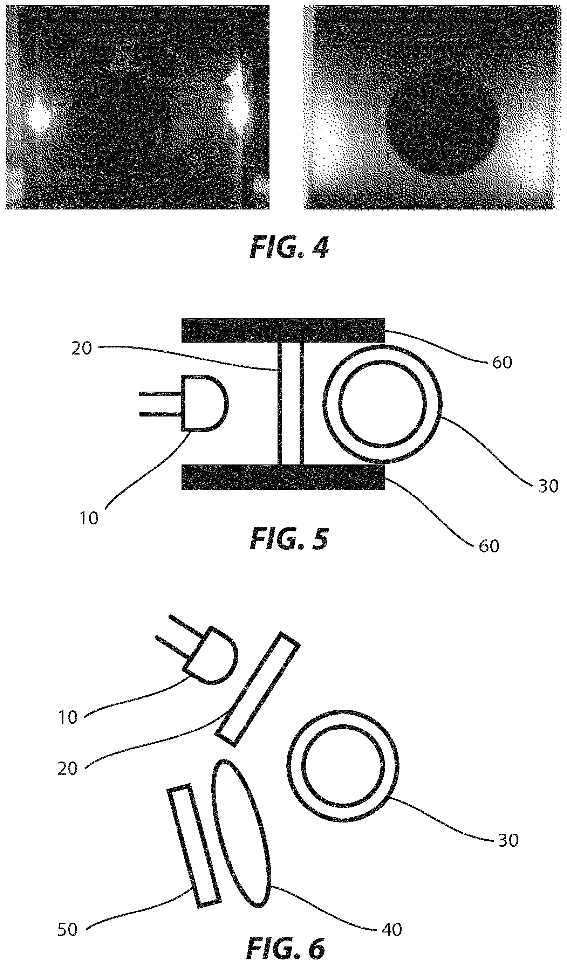

Fig. 4 zeigt veranschaulichend und schematisch eine Verbesserung der Ausleuchtung, die durch die Homogenisierungsvorrichtung 20 erhalten werden kann.

In einem Versuchsaufbau wurde der Schlauch oder Schlauchabschnitt 30 durch einen mit Wasser gefüllten Glaskolben von 5 cm Durchmesser repräsentiert, und wurde ein in einem Medium (hier Wasser) abzubildendes Objekt durch eine Kunststoffkugel von 1 cm Durchmesser repräsentiert.

Das linksseitige Teilbild in Fig. 4 zeigt eine Bitmap-Darstellung einer Aufnahme ohne Homogenisierungsvorrichtung 20, und das rechtsseitige Teilbild in Fig. 4 zeigt eine Bitmap-Darstellung einer Aufnahme mit der Homogenisierungsvorrichtung 20, versuchsweise in Form einer Diffusorfolie, zwischen der Lichtquelle 10 und dem Glaskolben (Schlauch oder Schlauchabschnitt 30).

Wie aus Fig. 4 unmittelbar ersichtlich ist, ist die Qualität der Ausleuchtung unter Verwendung der Homogenisierungsvorrichtung 20 deutlich erhöht. Denn während in dem linksseitigen Teilbild (ohne Homogenisierungsvorrichtung 20) das Licht als vor allem am Rand des Glaszylinders lokalisiert zu erkennen ist und die Ausleuchtung der Flüssigkeit und der Kunststoffkugel nicht ausreichen, um die Konturen der Kunststoffkugel gut identifizieren zu können, zeigt das rechtsseitige Teilbild (mit Homogenisierungsvorrichtung 20) einen breiten, gut ausgeleuchteten Streifen, vor dessen Hintergrund die Kontur der Kunststoffkugel hinreichend gut definiert festgelegt ist, um die Geometrie der Kugel vermessen zu können. Noch bestehende Helligkeitsunterschiede zwischen Rand und Mitte des Glaskolbens sind im Vergleich zu dem linksseitigen Teilbild deutlich verringert.

Die beste bzw. homogenste Ausleuchtung kann erzielt werden, wenn die Homogenisierungsvorrichtung 20 nächstmöglich an, d.h. so nahe wie möglich vor, dem Schlauch oder Schlauchabschnitt 30 angeordnet wird. Der Grund hierfür besteht darin, dass Homogenisierungsvorrichtungen für Licht in Form von beispielsweise Streuscheiben oder Diffusorfolien das Licht streuen und so eine möglichst homogene, aber auch isotrope Verteilung des Lichts nach der Homogenisierungsvorrichtung 20 erzeugen. Infolge dessen verringert sich die Beleuchtungsintensität mit zunehmendem Abstand von der Homogenisierungsvorrichtung 20, da auf Grund der Strahlungsisotropie die beleuchtete Fläche zunimmt. Gleichzeitig nimmt aber mit zunehmendem Abstand von der Homogenisierungsvorrichtung 20 auch die Homogenität der Bestrahlung vor allem in Randbereichen ab. Es wird angemerkt, dass in der Praxis ein erreichbarer kleinster Abstand von Homogenisierungsvorrichtung 20 und Schlauch oder Schlauchabschnitt 30 grundlegend auch durch eine in einer Kamera verwendete Optik und deren Öffnungswinkel und Fokusbereich bestimmt wird.

Fig. 5 zeigt ein zweites Ausführungsbeispiel mit einer Lichtleitvorrichtung 60, die dazu angeordnet ist, einer Verringerung der Beleuchtungsintensität nach der Homogenisierungseinrichtung 20 entgegenzuwirken. In diesem zweiten Ausführungsbeispiel ist die Lichtleitvorrichtung 60 in zumindest dem Bereich zwischen der Homogenisierungsvorrichtung 20 und dem Schlauch oder Schlauchabschnitt 30 als eine Licht reflektierende Wandung konfiguriert, welche zumindest den vorgenannten Bereich ummantelnd umgibt und welche an ihrer Innenseite das von der Lichtquelle 10 eingestrahlte Licht reflektiert. Auf diese Weise trifft auch Licht, das die Homogenisierungsvorrichtung 20 unter einem großen Winkel verlässt, nach seiner Reflektion an der Wandung auf den Schlauch oder Schlauchabschnitt 30. Die Lichtausbeute ist weiter dadurch steigerbar, dass sich die reflektierenden Wandungen der Lichtleitvorrichtung 60 bis über die Lichtquelle 10 erstrecken. In diesem Fall trifft auch bereits von der Lichtquelle 10 unter einem großen Winkel abgestrahltes Licht zunächst auf die Homogenisierungsvorrichtung 20 und danach auf den Schlauch oder Schlauchabschnitt 30. Insgesamt vorteilhaft erlauben Lichtleitvorrichtungen wie beispielsweise lichtreflektierende Wandungen eine beliebige Positionierung der Homogenisierungsvorrichtung 20, da auch das von der Homogenisierungsvorrichtung 20 unter einem Winkel gestreute Licht nicht verloren geht, sondern auf den Schlauch trifft.

Es wird angemerkt, dass konfigurationsbezogen und/oder abhängig von einem Anwendungsfall die Lichtleitvorrichtung 60 auch bzw. alternativ eine nicht reflektierende und/oder in einer vorbestimmten Farbgebung, beispielsweise schwarz, gehaltene Wandung ausbilden kann, mit welcher unerwünschte Reflexionen an etwa einer glattflächigen Oberfläche eines Schlauchs aus beispielsweise einem Kunststoffmaterial vermeidbar oder zumindest verringerbar sind

Fig. 6 zeigt vereinfacht und schematisch einen Aufbau einer Erfassungsvorrichtung zur lokal aufgelösten Analyse eines Mediums in einem Schlauch oder Schlauchabschnitt gemäß einem dritten Ausführungsbeispiel, das geometrisch für eine Reflexionsmessung konfiguriert ist;

Alternativ zu einer Transmissionskonfiguration wie vorstehend in Zusammenhang mit dem ersten Ausführungsbeispiel beschrieben ist es möglich, eine geometrische Anordnung für Reflexionsmessungen vorzusehen. In diesem Fall wird Licht erfasst, welches von dem Medium im Schlauch oder Schlauchabschnitt 30 reflektiert wird. Dabei kann das reflektierte Licht unter einem beliebigen Winkel gegenüber dem einfallenden Licht gemessen werden. Kleine Winkel sind dabei vorteilhaft, da das dorthin reflektierte Licht normalerweise die höchste Intensität aufweist. In der Reflexionskonfiguration ist darauf zu achten, dass die Homogenisierungsvorrichtung 20 das Sichtfeld des Detektorarrays möglichst nicht einschränkt, da andernfalls von dem Schlauch oder Schlauchabschnitt 30 reflektiertes Licht vor Erreichen des Detektorarrays 50 zunächst wieder die Homogenisierungsvorrichtung 20 durchlaufen muss und infolge dessen ein Informationsverlust auftreten kann.

In Abhängigkeit von dem Winkel zwischen einfallendem Licht und Detektionsrichtung sind mit der in Fig. 6 dargestellten Konfiguration außer einer Messung von reflektiertem Licht eine Vielzahl weiterer Messaufgaben durchführbar. Beispielsweise kann von dem Medium im Inneren des Schlauchs oder Schlauchabschnitts 30 gestreutes Licht gemessen werden. Dieses ist umso stärker, je inhomogener das Medium ist, und erlaubt aufgrund dessen Rückschlüsse auf vorhandene Verschmutzung oder Lufteinschlüsse. Ein optimaler Winkel zwischen Lichteinstrahlrichtung und Lichtdetektionsrichtung hängt von der Größe der zu vermessenden Elemente ab.

Ferner sind mit einer Konfiguration der vorgenannten Art auch abbildende Fluoreszenzmessungen und/oder Lumineszenzmessungen an dem Medium in dem Schlauch oder Schlauchabschnitt 30 durchführbar. Die Fluoreszenz oder Lumineszenz wird durch das eingestrahlte Licht angeregt. Ein in den Detektionspfad integrierter wellenlängenselektiver Filter (nicht dargestellt) ist dazu angeordnet, das Fluoreszenzlicht oder das Lumineszenzlicht durchzulassen, das Anregungslicht aber zurückzuhalten. Das Detektorarray erfasst in diesem Fall sodann nur das Fluoreszenzlicht oder das Lumineszenzlicht. Bei einer Messung dieser Art wird vorzugsweise der Winkel zwischen Einstrahlrichtung und Detektionsrichtung voreingestellt, bei dem die Intensität des Anregungslichts, das auf den Detektor trifft, minimal ist.

Wie vorstehend beschrieben wurde, stellt die Geometrie des Schlauchs oder Schlauchabschnitts 30 zusätzliche Herausforderungen für die homogene Ausleuchtung des Sichtfelds oder dar, und kann die Ausleuchtung generell durch den Einsatz der Homogenisierungsvorrichtung 20 und weiter durch eine spezielle Ausgestaltung oder Ausformung der Homogenisierungsvorrichtung 20 verbessert werden.

Insbesondere der Linseneffekt des Schlauchs oder Schlauchabschnitts 30 (vgl. Fig. 1) kann durch geometrisches Ausformen der Homogenisierungsvorrichtung 20 nach Art einer Streulinse kompensiert werden.

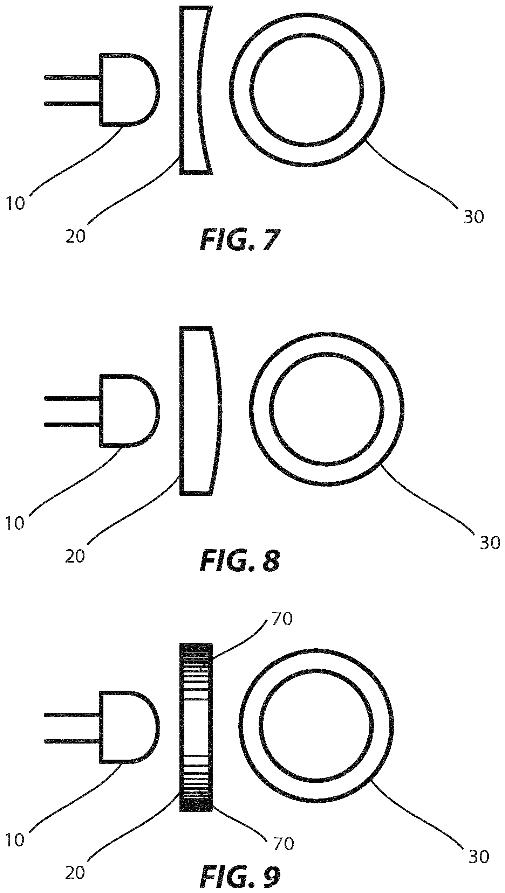

In einer Modifikation der vorstehend beschriebenen Ausführungsbeispiele wie in Fig. 7 gezeigt kann daher die Homogenisierungsvorrichtung 20 plano-konkav, d.h. mit einer planen Oberfläche auf einer der Lichtquelle 10 zugewandten Seite und einer konkaven Oberfläche auf einer dem Schlauch oder Schlauchabschnitt 30 zugewandten, gegenüberliegenden Seite ausgebildet sein. Vergleichbare Wirkungen sind mit anderen, zumindest eine konkave Oberfläche aufweisenden Formen, beispielsweise konkavkonkaven oder konkav-planen Ausführungsformen der Homogenisierungsvorrichtung 20, erzielbar. Mittels Bauraum einsparenden, konkaven Bauformen ist darüber hinaus auch die Homogenisierungsvorrichtung 20 möglichst nahe an dem Schlauch oder Schlauchabschnitt 30 platzierbar.

In einer weiteren Modifikation der vorstehend beschriebenen Ausführungsbeispiele wie in Fig. 8 gezeigt kann ferner die Homogenisierungsvorrichtung 20 plano-konvex, d.h. mit einer planen Oberfläche auf einer der Lichtquelle 10 zugewandten Seite und einer konvexen Oberfläche auf einer dem Schlauch oder Schlauchabschnitt 30 zugewandten, gegenüberliegenden Seite ausgebildet sein. In dem Fall einer plano-konvexen Form ist die Homogenisierungsvorrichtung 20 nach Art einer Fokussierlinse ausgestaltet und kann somit das homogenisierte Licht gezielt in einem bestimmten Bereich des Schlauchs oder Schlauchabschnitts 30 verstärkt werden. Vergleichbare Wirkungen sind mittels konvex-konvexen oder konvex-planen Formen erzielbar.

In einer nochmals weiteren Modifikation der vorstehend beschriebenen Ausführungsbeispiele wie in Fig. 9 gezeigt kann außerdem die Homogenisierungsvorrichtung 20 in ihrem Verlauf unterschiedlich absorbierend, beispielsweise mit erhöhter Absorption in einem Außenbereich oder Randbereich und geringerer Absorption in einem Mittenbereich ausgebildet sein. In Fig. 9 soll dies beispielsweise an oberen und unteren Kantenbereichen bei Betrachtung als Seitenansicht bzw. rechten und linken Kantenbereichen bei Betrachtung als Aufsicht angedeutet sein.

Unter Bezugnahme auf Fig. 2 führt das in Fig. 2 dargestellte Problem der ungleichen optischen Pfadlängen im Medium innerhalb des Schlauchs oder Schlauchabschnitts 30 genau dann zu einer ungleichmäßigen Ausleuchtung oder Beleuchtung des Detektorarrays 50, wenn das Medium im Schlauch oder Schlauchabschnitt 30 Licht absorbiert.

Eine solche unerwünschte Absorption kann mittels der Homogenisierungsvorrichtung 20 kompensiert oder zumindest verringert werden. Zu diesem Zweck kann die Homogenisierungsvorrichtung 20 nicht als reine und damit gleichförmig streuende Streuscheibe ausgebildet sein, sondern es können an vorbestimmten Stellen oder Bereichen der Homogenisierungsvorrichtung 20 einfallendes Licht absorbierende Stoffe als Absorber 70 beigefügt vorgesehen sein. Auf diese Weise kann das einfallende Licht an den vorbestimmten Stellen oder Bereichen gezielt (d.h. stärker oder schwächer gegenüber einer Bezugsregion der Homogenisierungsvorrichtung 20) absorbiert werden.

Werden diese Absorber 70 im Bereich beispielsweise der Oberkante und/oder der Unterkante der Homogenisierungsvorrichtung 20 eingebracht, wie schematisch in Fig. 9 angedeutet, wird einfallendes Licht an der Oberseite und/oder der Unterseite des Schlauchs oder Schlauchabschnitts 30 absorbiert und dadurch dort schwächer. Dadurch ist es möglich, das auf das Detektorarray 50 auftreffende Licht weiter zu homogenisieren, und kann ein Übersteuern und Übersprechen von Detektoren des Detektorarray 50, wie beispielsweise im linksseitigen Teilbild der Fig. 4 zu erkennen, verringert werden.

Im Einzelnen zeigt Fig. 9 eine Konfiguration der Homogenisierungsvorrichtung 20, bei welcher absorbierende Substanzen in einer Weise in die Homogenisierungseinrichtung 20 integriert sind, dass sich ein graduelles Konzentrationsprofil dieser Substanzen ergibt. In Fig. 9 ist dieses Konzentrationsprofil durch die unterschiedliche Querliniendichte entlang der Höhenrichtung der Homogenisierungseinrichtung 20 angedeutet. Danach ist in einem oberen und in einem unteren Bereich der Homogenisierungsvorrichtung 20 die Konzentration der beigefügten Substanzen jeweils höher und in einem zentralen Bereich oder Mittenbereich demgegenüber geringer. Wird das dadurch entstehende Lichtabsorptionsprofil geeignet an die geometrischen Gegebenheiten des Schlauchs oder Schlauchabschnitts 30 und die optischen Eigenschaften des Mediums angepasst, kann ein Übersprechen der Detektoren des Detektorarrays 50 an den Rändern des Detektorarrays 50 vermieden werden. Das Einbringen von Absorption in die Homogenisierungsvorrichtung 20 kann durch gezielte Veränderung des Basismaterials und/oder durch Kombination unterschiedlicher Materialien, beispielsweise durch Vorsehen einer Absorptionsfolie mit einem vorbestimmten Absorptionsprofil, die auf die Homogenisierungsvorrichtung aufgebracht oder aufgeklebt wird, erfolgen.

Eine vergleichbare Wirkung kann unter Verwendung eines Stufenprofils der Absorption in der Homogenisierungsvorrichtung 20 erzielt werden. Dabei kann durch entsprechende Veränderung des Materials an geeigneter Stelle und/oder durch Hinzufügen von absorbierendem Material in den Randbereichen eine Absorption nur in den oberen und unteren Randbereichen der Homogenisierungsvorrichtung 20 erzeugt sein.

Wie vorstehend beschrieben wurde, sind erfindungsgemäß Inhomogenitäten in einem von einem flüssigen Medium durchflossenen Schlauch oder Schlauchabschnitt mittels einer orts- und zeitaufgelösten Detektion der Inhomogenitäten auflösbar.

Insbesondere bei der Rückführung von Blut zu einem Patienten, etwa in Dialyseanwendungen, ist es zu vermeiden, dem Patienten Luft zu injizieren. Daher weisen Dialysemaschinen in der Regel Sensoren auf, die Luft in ihrem venösen Blutschlauchsystem erkennen und eine laufende Therapie anhalten, falls Luft detektiert wird. Ein ortsaufgelöstes Verfahren ist an dieser Stelle dahingehend vorteilhaft, dass das Luftvolumen in den Luftblasen genauer quantifizierbar und dadurch ein Patientenrisiko besser einschätzbar ist. Luftblasen in Blut sind Inhomogenitäten, welche weniger stark Licht absorbieren als das Blut selbst, und welche gleichzeitig einen geringen Brechungsindex haben. Mit Hilfe dieser beiden Eigenschaften und bei Wahl einer geeigneten Wellenlänge für das einfallende Licht, vorzugsweise im infraroten Spektralbereich, ist durch ein optisches Detektionssystem wie vorstehend beschrieben eine Detektion von Luftblasen in Blut möglich und sind Luftblasen nachweisbar. Ferner unterscheiden sich Blutgerinnsel von flüssigem Blut durch ihre Absorption, ihren Brechungsindex und die daraus folgende Lichtstreuung. Aus diesem Grund können Blutgerinnsel mit Hilfe von ortsaufgelösten optischen Messungen erkannt und quantifiziert werden. Kritisch für den Patienten ist, wenn sich ein Blutgerinnsel, das sich im Schlauchsystem oder Dialysator ausbildet, löst und über die venöse Leitung in den Patienten gelangt. Durch ein optisches Detektionssystem wie vorstehend beschrieben ist ein durch die venöse Leitung wanderndes oder fließendes Blutgerinnsel erkennbar, und kann dessen Zuführung zum Patienten vermieden werden.

Das optische Detektionssystem wie vorstehend beschrieben ist darüber hinaus zur Detektion von Verunreinigungen in NaCl- Lösung anwendbar. Gelegentlich kommt es auch bei aller Sorgfalt vor, dass Verunreinigungen aus dem Herstellungsprozess in einem Schlauchsystem oder Dialysator verbleiben. Diese Verunreinigungen werden beim Spülen des Schlauchsystems und des Dialysator gelöst und nach und nach ausgespült. Solche Verunreinigungen, die häufig in Form von Kunststofffasern oder Kunststoffflocken vorliegen, können aufgrund ihres höheren Brechungsindex gegenüber dem umgebenden Wasser mit orts- und zeitaufgelöster optischer Messung erkannt werden. Werden solche Verunreinigungen während des Spülvorgangs erkannt, kann eine Vergrößerung des Spülvolumens ein vollständiges Ausspülen dieser Verunreinigungen erreichen und damit vermeiden, dass dem Patienten während der Therapie Fremdstoffe zugeführt werden.

Ebenso ist es möglich, dass aufgrund von Gegebenheiten während einer Therapie Verunreinigungen innerhalb des Schlauchsystems produziert werden, z.B. durch Abrieb an der Innenseite des Pumpsegments. Auch diese Verunreinigungen können aufgrund ihrer von Blut abweichenden optischen Eigenschaften erkannt werden, so dass deren Zuführung zum Patienten verhindert werden kann.

Wie vorstehend beschrieben und experimentell nachgewiesen wurde, können durch eine homogenere Ausleuchtung des Bildfelds im Schlauch für eine abbildende/ortsaufgelöste Sensorik in einem eine Erfassungsvorrichtung für ein Medium in einem Schlauchabschnitt bildenden optischen Detektionssystem wie vorstehend beschrieben ein verbesserter Nachweis von Lufteinschlüssen oder Verunreinigungen in dem in dem Schlauch geführten Medium (hier Flüssigkeit) geführt und eine verbesserte Identifizierung und Vermessung der Geometrie von Lufteinschlüssen oder Verunreinigungen in dem Medium erhalten werden.

Erzielbar ist dies erfindungsgemäß durch eine Erfassungsvorrichtung für ein Medium in einem Schlauchabschnitt mit einer abbildenden Optik, einem Schlauchabschnitt, zumindest einer Lichtquelle, die an einer ersten Position in Bezug auf einen von Licht durchstrahlbaren Schlauchabschnitt angeordnet und dazu konfiguriert ist, Licht auf den Schlauchabschnitt abzustrahlen, einer Detektoreinrichtung, die an einer zweiten Position in Bezug auf den Schlauchabschnitt angeordnet und dazu konfiguriert ist, durch den Schlauchabschnitt hindurch gelangtes Licht von der zumindest einen Lichtquelle zu empfangen und ein Medium in dem Schlauchabschnitt ortsauflösend zu analysieren, und einer Homogenisierungsvorrichtung, die an einer Position zwischen der zumindest einen Lichtquelle und dem Schlauchabschnitt angeordnet und dazu konfiguriert ist, zur Erfassung und Quantisierung von Helligkeitsunterschieden aufgrund lokaler Unterschiede des Mediums in dem Schlauchabschnitt eine homogene und/oder isotrope Verteilung des Lichts von der zumindest einen Lichtquelle zu erzeugen, bevor das Licht in den Schlauchabschnitt eintritt, wobei die Homogenisierungsvorrichtung entlang einer Richtung senkrecht zu der Durchstrahlungsrichtung in vorbestimmte Bereiche der Homogenisierungsvorrichtung durch gezielte Veränderung des Basismaterials und/oder durch Kombination unterschiedlicher Materialien vorbestimmte Konzentrationen Licht absorbierender Substanzen aufweist, die eine veränderlich Licht absorbierende Funktion der Homogenisierungsvorrichtung bereitstellen,dadurch gekennzeichnet, dass die Homogenisierungsvorrichtung in einem durch zumindest die abbildende Optik, deren Öffnungswinkel und deren Fokusbereich bestimmten kleinsten Abstand zu dem Schlauchabschnitt angeordnet ist; eine Lichtleitvorrichtung, die eine erste und eine zweite Lichtleitvorrichtung umfasst und die in zumindest dem Bereich zwischen der Homogenisierungsvorrichtung und dem Schlauchabschnitt als eine Wandung konfiguriert ist, welche zumindest den Bereich ummantelnd umgibt, wobei die zumindest eine Lichtquelle und/oder die Homogenisierungsvorrichtung zwischen der ersten und der zweiten Lichtleitvorrichtung angeordnet ist/sind; und die vorbestimmten Konzentrationen Licht absorbierender Substanzen in die Homogenisierungsvorrichtung derart integriert sind, dass in einem oberen und in einem unteren Außenbereich der Homogenisierungsvorrichtung die Konzentration der beigefügten Substanzen jeweils höher und in einem zentralen Bereich oder Mittenbereich demgegenüber geringer ist, wobei der obere und der untere Außenbereich der Homogenisierungsvorrichtung sowie deren zentraler Bereich oder Mittenbereich entlang der Richtung senkrecht zu der Durchstrahlungsrichtung angeordnet sind, und ein dadurch entstehendes Lichtabsorptionsprofil an geometrische Gegebenheiten des Schlauchabschnitts und die optischen Eigenschaften des Mediums angepasst ist.

Die Erfindung wurde vorstehend anhand bevorzugter Ausführungsbeispiele beschrieben.

**Patentansprüche**

1. Erfassungsvorrichtung für ein Medium in einem Schlauchabschnitt (30), beinhaltend:

eine abbildende Optik;
den Schlauchabschnitt (30);
zumindest eine Lichtquelle (10), die an einer ersten Position in Bezug auf den von Licht durchstrahlbaren Schlauchabschnitt (30) angeordnet und dazu konfiguriert ist, Licht auf den Schlauchabschnitt (30) abzustrahlen;
eine Detektoreinrichtung (50), die an einer zweiten Position in Bezug auf den Schlauchabschnitt (30) angeordnet und dazu konfiguriert ist, durch den Schlauchabschnitt (30) hindurch gelangtes Licht von der zumindest einen Lichtquelle (10) zu empfangen und ein Medium in dem Schlauchabschnitt (30) ortsauflösend zu analysieren; und
eine Homogenisierungsvorrichtung (20), die an einer Position zwischen der zumindest einen Lichtquelle (10) und dem Schlauchabschnitt (30) angeordnet und dazu konfiguriert ist, zur Erfassung und Quantisierung von Helligkeitsunterschieden aufgrund lokaler Unterschiede des Mediums in dem Schlauchabschnitt (30) eine homogene und/oder isotrope Verteilung des Lichts von der zumindest einen Lichtquelle (10) zu erzeugen, bevor das Licht in den Schlauchabschnitt (30) eintritt, wobei die Homogenisierungsvorrichtung (20) entlang einer Richtung senkrecht zu der Durchstrahlungsrichtung in vorbestimmte Bereiche der Homogenisierungsvorrichtung (20) durch gezielte Veränderung des Basismaterials und/oder durch Kombination unterschiedlicher Materialien vorbestimmte Konzentrationen Licht absorbierender Substanzen aufweist, die eine veränderlich Licht absorbierende Funktion der Homogenisierungsvorrichtung (20) bereitstellen,

**dadurch gekennzeichnet, dass**

die Homogenisierungsvorrichtung (20) in einem durch zumindest die abbildende Optik, deren Öffnungswinkel und deren Fokusbereich bestimmten kleinsten Abstand zu dem Schlauchabschnitt (30) angeordnet ist; eine Lichtleitvorrichtung (60), die eine erste und eine zweite Lichtleitvorrichtung (60) umfasst und die in zumindest dem Bereich zwischen der Homogenisierungsvorrichtung (20) und dem Schlauchabschnitt (30) als eine Wandung konfiguriert ist, welche zumindest den Bereich ummantelnd umgibt, wobei die zumindest eine Lichtquelle (10) und/oder die Homogenisierungsvorrichtung (20) zwischen der ersten und der zweiten Lichtleitvorrichtung (60) angeordnet ist/sind; und

die vorbestimmten Konzentrationen Licht absorbierender Substanzen in die Homogenisierungsvorrichtung (20) derart integriert sind, dass in einem oberen und in einem unteren Außenbereich der Homogenisierungsvorrichtung (20) die Konzentration der beigefügten Substanzen jeweils höher und in einem zentralen Bereich oder Mittenbereich demgegenüber geringer ist, wobei der obere und der untere Außenbereich der Homogenisierungsvorrichtung (20) sowie deren zentraler Bereich oder Mittenbereich entlang der Richtung senkrecht zu der Durchstrahlungsrichtung angeordnet sind, und ein dadurch entstehendes Lichtabsorptionsprofil an geometrische Gegebenheiten des Schlauchabschnitts (30) und die optischen Eigenschaften des Mediums angepasst ist.

2. Erfassungsvorrichtung nach Anspruch 1, bei der eine Absorptionsfolie mit einem vorbestimmten Absorptionsprofil, die auf die Homogenisierungsvorrichtung aufgebracht ist, dazu angeordnet ist, die vorbestimmten Konzentrationen Licht absorbierender Substanzen zu erzeugen.

3. Erfassungsvorrichtung nach Anspruch 1 oder 2, bei der in einer geometrischen Anordnung für eine Transmissionsmessung die zumindest eine Lichtquelle (10) derart auf einer ersten Seite des Schlauchabschnitts (30) angeordnet ist und die Detektoreinrichtung (50) derart auf einer zweiten Seite des Schlauchabschnitts (30) angeordnet ist, dass sich der Schlauchabschnitt (30) zwischen der zumindest einen Lichtquelle (10) und der Detektoreinrichtung (50) befindet.

4. Erfassungsvorrichtung nach Anspruch 1, bei der in einer geometrischen Anordnung für eine Reflexionsmessung die zumindest eine Lichtquelle (10) und die Detektoreinrichtung (50) bezüglich des Schlauchabschnitts (30) auf derselben Seite angeordnet sind und dabei einen vorbestimmten Winkel zueinander einnehmen.

5. Erfassungsvorrichtung nach einem der vorangehenden Ansprüche, bei der die Homogenisierungsvorrichtung (20) eine Streuplatte, eine Diffusorfolie oder ein transparentes Medium mit aufgerauter Oberfläche ist.

6. Erfassungsvorrichtung nach einem der vorangehenden Ansprüche, bei der die abbildende Optik vor der Detektoreinrichtung (50) und/oder zwischen der zumindest einen Lichtquelle (10) und der Homogenisierungsvorrichtung (20) angeordnet ist.

7. Erfassungsvorrichtung nach einem der vorangehenden Ansprüche, bei der eine Größe der Homogenisierungsvorrichtung (20) mit zumindest der Größe eines Sichtfelds der Detektoreinrichtung (50) übereinstimmt.

8. Erfassungsvorrichtung nach einem der vorangehenden Ansprüche, bei der die zumindest eine Lichtquelle (10) dazu konfiguriert ist, eine Ausdehnung der Homogenisierungsvorrichtung (20) in Längsrichtung des Schlauchabschnitts (30) vollständig auszuleuchten, wobei für eine vollständige Ausleuchtung eine dazu angepasste Form der zumindest einen Lichtquelle (10) oder eine Vielzahl von Lichtquellenelementen und/oder strahlformende Elemente in einem Lichtweg vorgesehen sind.

9. Medizinisches Gerät mit einer Erfassungsvorrrichtung nach einem der vorangehenden Ansprüche, wobei das medizinische Gerät als eine Maschine zur extrakorporalen Blutbehandlung ausgebildet ist.

**Claims**

1. A detecting device for a medium inside a tube portion (30), comprising:

   an imaging optical system;
   the tube portion (30);
   at least one light source (10) being arranged at a first position relative to the tube portion (30) adapted to be transilluminated with light and being configured to irradiate light onto the tube portion (30);
   a detector unit (50) being arranged at a second position relative to the tube portion (30) and being configured to receive light from the at least one light source (10) passed through the tube portion (30) and to analyze a medium inside the tube portion (30) in a spatially resolving manner; and
   a homogenizing device (20) being arranged at a position between the at least one light source (10) and the tube portion (30) and being configured, for detecting and quantifying differences in brightness due to local differences of the medium inside the tube portion (30), to create a homogenous and/or isotropic distribution of the light from the at least one light source (10) before the light enters into the tube portion (30), wherein the homogenizing device (20) comprises predetermined concentrations of light-absorbing substances, which provide a variably light-absorbing function of the homogenizing device (20), along a direction perpendicularly to the transilluminating direction in predetermined areas of the homogenizing device (20),
   **characterized in that**
   the homogenizing device (20) is arranged at a smallest distance from the tube portion (30) determined by at least the imaging optical system, its opening angle and its focal range;
   a light conducting device (60) comprising a first and a second light conducting device (60) and being configured in at least the area between the homogenizing device (20) and the tube portion (30) as a circumferential wall surrounding at least the area in a sheathing manner, wherein the at least one light source (10) and/or the homogenizing device (20) is/are arranged between the first and the second light conducting device (60); and the predetermined concentrations of light-absorbing substances are integrated in the homogenizing device (20) in such a manner that in an upper and in a lower outer area of the homogenizing device (20), the concentration of the added substances is respectively higher and in a central area or middle area, in contrast, lower, wherein the upper and the lower outer area of the homogenizing device (20) as well as its central area or middle area are arranged along the direction perpendicular to the transilluminating direction, and a light absorption profile resulting therefrom is adapted to geometrical conditions of the tube portion (30) and the optical properties of the medium.

2. Detecting device according to claim 1, wherein an absorption film with a predetermined absorption profile applied to the homogenizing device is arranged to generate the predetermined concentrations of light absorbing substances.

3. The detecting device according to claim 1 or 2, wherein in a geometric arrangement for transmission measurement the at least one light source (10) is arranged on a first side of the tube portion (30) and the detector unit (50) is arranged on a second side of the tube portion (30) so that the tube portion (30) is located between the at least one light source (10) and the detector unit (50).

4. The detecting device according to claim 1, wherein in a geometric arrangement for reflection measurement, the at least one light source (10) and the detector unit (50) are arranged on the same side with respect to the tube portion (30) and accordingly adopt a predetermined angle relative to each other.

5. The detecting device according to one of the preceding claims, wherein the homogenizing device (20) is a scattering disk, a diffusor film or a transparent medium having a roughened surface.

6. The detecting device according to one of the preceding claims, wherein the imaging optical system is arranged upstream of the detector unit (50) and/or between the at least one light source (10) and the homogenizing device (20).

7. The detecting device according to one of the preceding claims, wherein a size of the homogenizing device (20) is in conformity with at least the size of a field of view of the detector unit (50).

8. The detecting device according to one of the preceding claims, wherein the at least one light source (10) is configured to completely illuminate an expansion of the homogenizing device (20) in the longitudinal direction of the tube portion (30), wherein for complete illumination an appropriately adapted shape of the at least one light source (10) or a plurality of light source elements and/or beam-forming elements are provided in a light path.

9. A medical apparatus comprising a detecting device according to one of the preceding claims, wherein the medical apparatus is in the form of a machine for extracorporeal blood treatment.

**Revendications**

1. Dispositif de détection destiné à un milieu dans une section de flexible (30), incluant :

    une optique d'imagerie ;
    la section de flexible (30) ;
    au moins une source de lumière (10) qui est agencée au niveau d'une première position par rapport à la section de flexible (30) pouvant être traversée par de la lumière, et qui est configurée pour émettre de la lumière sur la section de flexible (30) ;
    un appareil de détection (50) qui est agencé au niveau d'une seconde position par rapport à la section de flexible (30) et qui est configuré pour recevoir de la lumière de la au moins une source de lumière (10) après qu'elle a traversé la section de flexible (30), et pour analyser en résolution spatiale un milieu dans la section de flexible (30) ; et
    un dispositif d'homogénéisation (20) qui est agencé au niveau d'une position entre la au moins une source de lumière (10) et la section de flexible (30) et qui est configuré pour générer une répartition homogène et/ou isotrope de la lumière de la au moins une source de lumière (10) afin de détecter et quantifier des différences de luminosité dues à des différences locales du milieu au sein de la section de flexible (30), avant que la lumière n'entre dans la section de flexible (30), dans lequel le dispositif d'homogénéisation (20) présente le long d'une direction perpendiculaire à la direction d'irradiation dans des régions prédéterminées du dispositif d'homogénéisation (20) au moyen d'une modification ciblée du matériau de base et/ou au moyen d'une combinaison de différents matériaux des concentrations prédéterminées de substances absorbant de la lumière qui fournissent une fonction variable absorbant de la lumière du dispositif d'homogénéisation (20),
    **caractérisé en ce que**
    le dispositif d'homogénéisation (20) est agencé à une distance la plus petite possible de la section de flexible (30), distance qui est déterminée au moyen d'au moins l'optique d'imagerie, son angle d'ouverture et sa plage de mise au point ;
    un dispositif de guidage de lumière (60) qui comprend des premier et second dispositifs de guidage de lumière (60) et qui est configuré dans au moins la région entre le dispositif d'homogénéisation (20) et la section de flexible (30) comme une paroi qui entoure de manière enveloppante au moins ladite région, dans lequel la au moins une source de lumière (10) et/ou le dispositif d'homogénéisation (20) est/sont agencé(s) entre les premier et second dispositifs de guidage de lumière (60) ; et
    les concentrations prédéterminées de substances absorbant de la lumière sont intégrées dans le dispositif d'homogénéisation (20) de telle manière que la concentration des substances ajoutées est respectivement plus élevée dans une région extérieure supérieure et dans une région extérieure inférieure du dispositif d'homogénéisation (20) et est au contraire plus faible dans une région centrale ou une région médiane, dans lequel les régions extérieures supérieure et inférieure du dispositif d'homogénéisation (20) ainsi que leur région centrale ou région médiane sont agencées le long de la direction perpendiculaire à la direction d'irradiation, et un profil d'absorption de lumière en résultant est adapté aux conditions géométriques de la section de flexible (30) et aux propriétés optiques du milieu.

2. Dispositif de détection selon la revendication 1, dans lequel un film d'absorption avec un profil d'absorption prédéterminé qui est appliqué au dispositif d'homogénéisation est agencé afin de générer les concentrations prédéterminées de substances absorbant de la lumière.

3. Dispositif de détection selon la revendication 1 ou 2, dans lequel, dans un agencement géométrique destiné à une mesure de transmission, la au moins une source de lumière (10) est agencée sur un premier côté de la section de flexible (30) et l'appareil de détection (50) est agencé sur un second côté de la section de flexible (30) de telle manière que la section de flexible (30) se trouve entre la au moins une source de lumière (10) et l'appareil de détection (50).

4. Dispositif de détection selon la revendication 1, dans lequel, dans un agencement géométrique destiné à une mesure de réflexion, la au moins une source de lumière (10) et l'appareil de détection (50) sont agencés du même côté par rapport à la section de flexible (30) et adoptent ainsi un angle prédéterminé l'un par rapport à l'autre.

**5.** Dispositif de détection selon l'une quelconque des revendications précédentes, dans lequel le dispositif d'homogénéisation (20) est une plaque de diffusion, un film diffuseur ou un milieu transparent à surface rugueuse.

**6.** Dispositif de détection selon l'une quelconque des revendications précédentes, dans lequel l'optique d'imagerie est agencée devant l'appareil de détection (50) et/ou entre la au moins une source de lumière (10) et le dispositif d'homogénéisation (20).

**7.** Dispositif de détection selon l'une quelconque des revendications précédentes, dans lequel une taille du dispositif d'homogénéisation (20) correspond à au moins la taille d'un champ de vision de l'appareil de détection (50).

**8.** Dispositif de détection selon l'une quelconque des revendications précédentes, dans lequel la au moins une source de lumière (10) est configurée pour éclairer complètement une extension du dispositif d'homogénéisation (20) dans la direction longitudinale de la section de flexible (30), dans lequel une forme appropriée de la au moins une source de lumière (10) ou une pluralité d'éléments formant source de lumière et/ou d'éléments de mise en forme de faisceau est/sont fourni(s) sur un trajet lumineux en vue d'un éclairage complet.

**9.** Appareil médical avec un dispositif de détection selon l'une quelconque des revendications précédentes, dans lequel l'appareil médical est réalisé sous la forme d'une machine de traitement extracorporel du sang.

**FIG. 1**

30

**FIG. 2**

30

10

20

30

40

50

**FIG. 3**

**FIG. 4**

**FIG. 5**

**FIG. 6**

10

20

30

**FIG. 7**

10

20

30

**FIG. 8**

70

10

20

70

30

**FIG. 9**

**IN DER BESCHREIBUNG AUFGEFÜHRTE DOKUMENTE**

**In der Beschreibung aufgeführte Patentdokumente**

- EP 2264512 A1